# EUROPEAN PATENT APPLICATION

(11) **EP 2 519 075 A1**
(43) Date of publication of application: **31.10.2012**
(21) Application number: 11008280.7
(22) Date of filing: 13.10.2011
(51) Int. Cl.: H05B 3/34, H05B 3/56

(54) **Continuous type heating pad**

(30) Priority: 25.04.2011 TW 100207280
(71) Applicant: Hsin Yung Chien Co., Ltd., Nantou (TW)
(72) Inventor: Lin, Chi-Chin, Nan Kung Industrial Zone Nantou (TW)
(74) Representative: Banzer, Hans-Jörg

(57) **Abstract**

A continuous type heating pad includes a laminated body (200) made from first and second cover layers (10, 20), and an electric heating unit (30). Each of the first and second cover layers (10, 20) has a width of 500 mm - 4200 mm, and includes an electrical insulation material (11, 21) embedded in a silicone rubber material (120, 220). The electric heating unit (30) is disposed between the first and second cover layers (10, 20) when the first and second cover layers pass through lamination rollers (40) and a vulcanizing drum (101) to form a laminated body (200), which is then rolled up to form a rolled laminated body (300).

## Description

This invention relates to a thermoelectric device, more particularly to a continuous type heating pad.

Flexible heating pads have been widely used in small size warming articles, such as, warming clothes, warming seats, warming backrests, warming blankets, etc., and large-size warming devices, such as floor warming devices, snow-melting and warming devices, etc., which are commonly used in cold zone areas.

Conventional heating pads generally include a pair of cover layers sandwiching an electric heating unit therebetween. Each cover layer includes an electrical insulation cloth enveloped by an unvulcanized silicone material. Such heating pads are usually fabricated by integrating the cover layers with the electric heating unit using a molding device and by heating the molding device to cure or vulcanize the silicone material. However, when the size of the heating pad is large, a large size molding device is needed for manufacturing, which can result in a reduction of manufacturing efficiency, an increased energy consumption, and an increase of the equipment and manufacturing costs.

A main object of the present invention is to provide a continuous type heating pad that can be manufactured continuously and easily and that can be tailored to a certain size as desired.

Accordingly, a continuous type heating pad of this invention includes a continuously extending laminated body made from first and second cover layers, and an electric heating unit. The first cover layer extends continuously along a length direction of the laminated body, and having a width of 500 mm - 4200 mm along a width direction of the laminated body. The first cover layer has a first electrical insulation material embedded in a first silicone rubber material, and first inner and outer surfaces. The second cover layer extends continuously along the length direction and havingawidthof 500 mm - 4200 mm along the width direction . The second cover layer has a second electrical insulation material embedded in a second silicone rubber material, and second inner and outer surfaces. The second cover layer is bonded vulcanizedly to the first cover layer such that the first and second inner surfaces confront each other. The electric heating unit is disposed between the first and second inner surfaces and distributed throughout the length of the laminated body.

Other features and advantages of the present invention will become apparent in the following detailed description of the preferred embodiments with reference to the accompanying drawings, of which:
Fig. 1 is an elevation view of a continuous type heating pad according to a first preferred embodiment of the present invention;
Figure 2 is a top plan view of the heating pad in the first preferred embodiment;
Fig. 3 is a fragmentary sectional view of the heating pad in the first preferred embodiment;
Figure 4 is a schematic view illustrating a manufacturing process for the heating pad; and
Figure 5 is a top plan view of a heating pad according to a second preferred embodiment of the present invention;

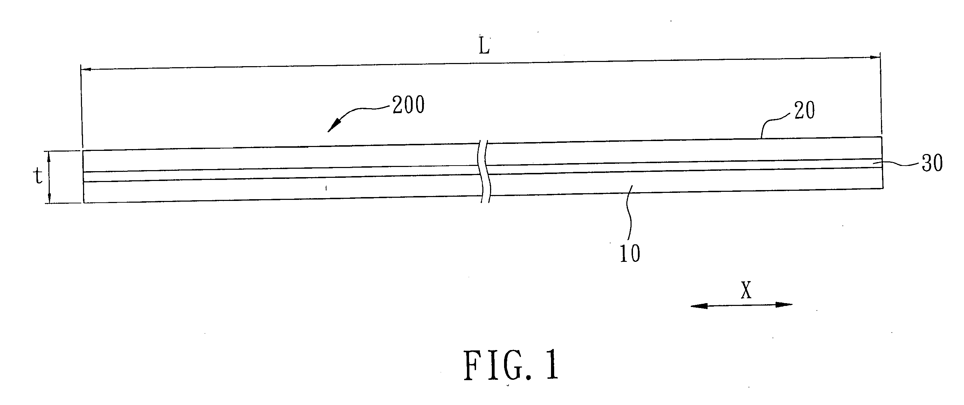

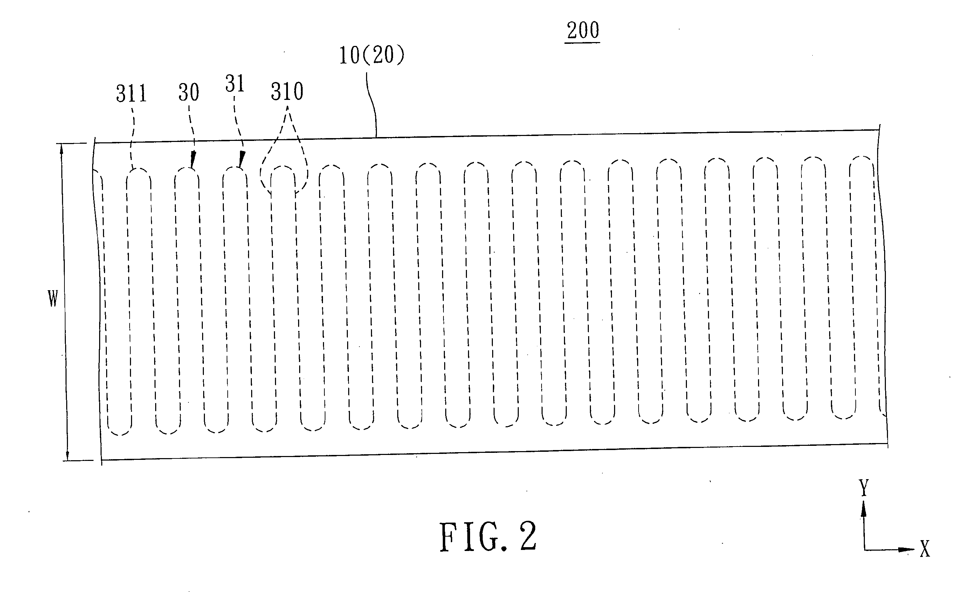

Referring to Figures 1, 2 and 3, according the first preferred embodiment of the present invention, a continuous type heating pad includes a continuously extending laminated body 200 made from a first cover layer 10, a second cover layer 20, and an electric heating unit 30.

The first cover layer 10 extends longitudinally along a length direction (X) of the laminated body 200, and has a width of 500 mm - 4200 mm along a width direction (Y). The first cover layer 10 has a first electrical insulation material 11 embedded in a first silicone rubber material 120, and first inner and outer surfaces 121, 122.

The second cover layer 20 extends longitudinally along the length direction (X), and has a width of 500 mm - 4200 mm along the width direction (Y). The second cover layer 20 includes a second electrical insulation material 21 embedded in a second silicone rubber material 220, and second inner and outer surfaces 221, 222. The second cover layer 20 is bonded vulcanizedly to the first cover layer 10 such that the first and second inner surfaces 121, 221 confront each other.

In this embodiment, both of the first electrical insulation material 11 and the second electrical insulation material 21 are made from a glass fiber cloth.

The electric heating unit 30 is disposed between the first and second inner surfaces 121, 221 distributed throughout the length of the laminated body 200. The electric heating unit 30 includes a continuously extending electric heating element 31 having a constant configuration throughout the length of the laminated body 200. In this embodiment, the electric heating element 31 is an electric heating wire, which is bent alternately in opposite directions to form a plurality of spaced apart line sections 310 interconnected by U-shaped turns 311. However, instead of the wire-type heating element 31, a strip-type heating element may be used for the electric heating unit 30.

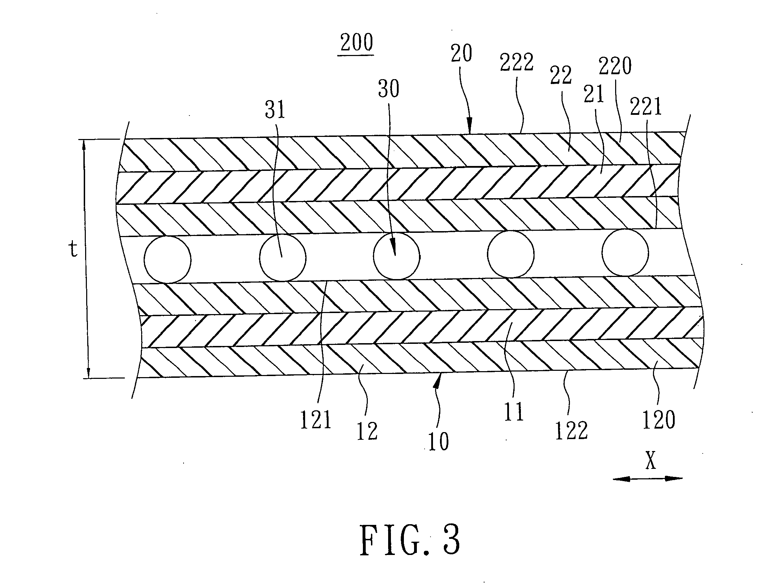

Referring to Figure 3, a thickness (t) measured from the first outer surface 122 to the second outer surface 222 is preferably about 0.4 mm-10 mm.

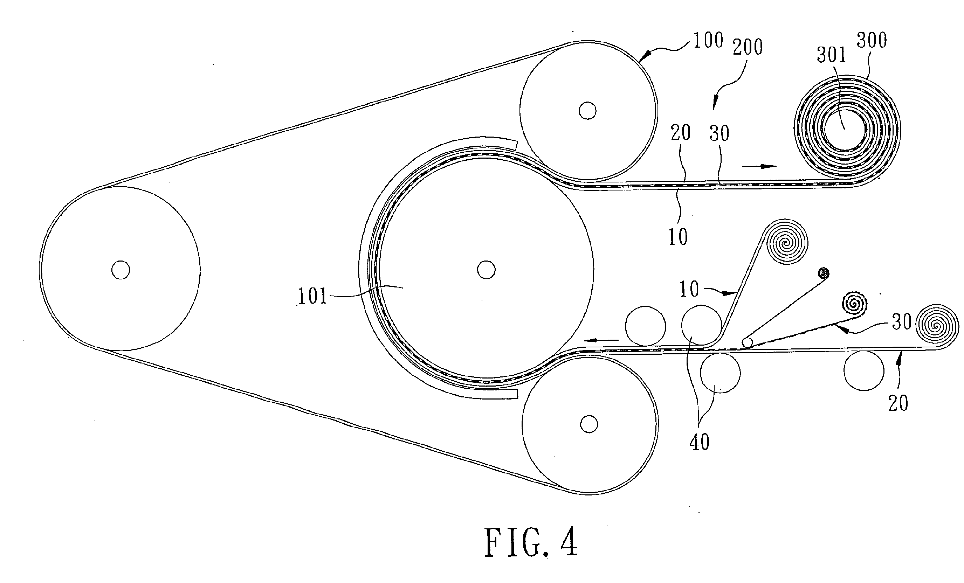

Referring to Figure 4, the continuous type heating pad according to the present invention may be fabricated as follows:
Firstly, the first and second cover layers 10, 20 are advanced through a set of lamination rollers 40 to a vulcanizing machine 100 including a vulcanizing drum 101. The electric heating unit 30 is moved between the first and second cover layers 10, 20 while the first and second cover layers 10, 20 are passed through the lamination rollers 40 and the vulcanizing drum 101. After passing through the vulcanizing drum 101, the first and second silicone rubber materials 120, 220 are vulcanized so that the first and second cover layers 10, 20 are bonded to each other to form the laminated body 200. Finally, the laminated body 200 is rolled up to form a rolled laminated body 300, which includes a core 301. The laminated body 200 is wound around the core 301. Preferably, the laminated body (200) is at least 3 m long. The rolled laminated body 300 may be unrolled and tailored to form a plurality of individual heating pad elements having different desired sizes.

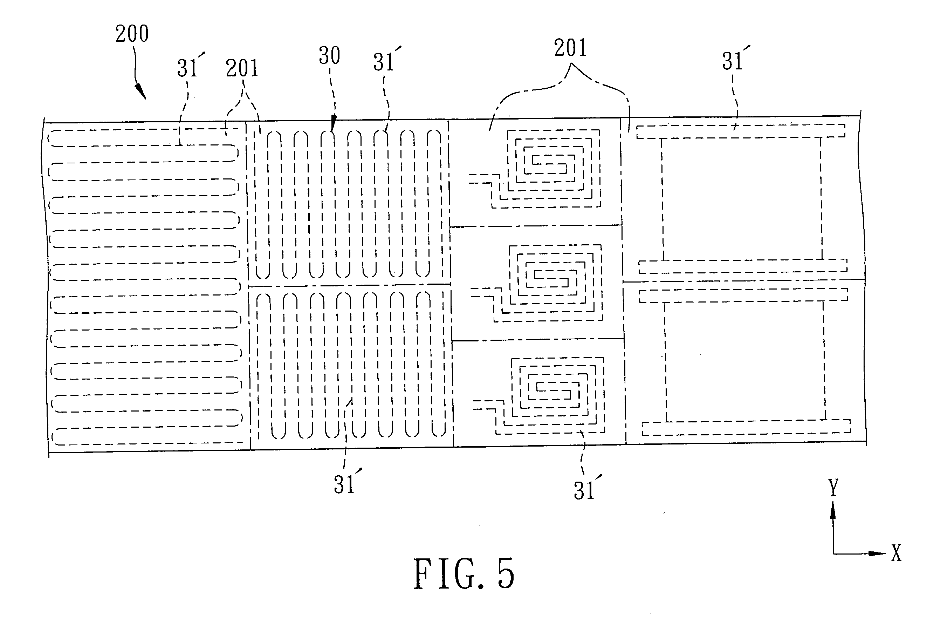

Referring to Figure 5, a continuous type heating pad according to a second preferred embodiment of this invention is generally identical to the first preferred embodiment. However, the laminated body 200 is divided into a plurality of regions 201, and the electric heating unit 30' includes a plurality of electric heating elements 31' that are separated from each other and that are disposed respectively in the regions 201. The electric heating elements 31' have different configurations and are arranged side by side along the length direction (X) and the width direction (Y). The laminated body 200 in this embodiment may be tailored readily by cutting and separating the regions 201.

## Claims

1. A continuous type heating pad, comprising:
a continuously extending laminated body (200) made from first and second cover layers (10, 20), and an electric heating unit (30);
said first cover layer (10) extending continuously along a length direction (x) of said laminated body, and having a width of 500 mm - 4200 mm along a width direction (Y) of said laminated body, said first cover layer (10) having a first electrical insulation material (11) embedded in a first silicone rubber material (120), and first inner and outer surfaces (121, 122);
a second cover layer (20) extending continuously along the length direction (X) and having a width of 500 mm - 4200 mm along the width direction (Y), said second cover layer (20) having a second electrical insulation material (21) embedded in a second silicone rubber material (220), and second inner and outer surfaces (221, 222) , said second cover layer (20) being bonded vulcanizedly to said first cover layer (10) such that said first and second inner surfaces (121, 221) confront each other; and
an electric heating unit (30) disposed between said first and second inner surfaces (121, 221) and distributed throughout a length of said laminated body (200).

2. The continuous type heating pad of Claim 1, wherein a thickness (t) measured from said first outer surface (122) to said second outer surface (222) is about 0.4 mm-10 mm.

3. The continuous type heating pad of Claim 1 or Claim 2, wherein said electric heating unit (30) includes an electric heating element (31) having a constant configuration that extends continuously along the length direction (X) of said laminated body (200).

4. The continuous type heating pad of any one of Claims 1-3, wherein said laminated body (200) is divided into a plurality of regions (201) , and said electric heating unit (30) includes a plurality of electric heating elements (31') that are separated from each other and that are disposed respectively in said regions (201).

5. The continuous type heating pad of Claim 4, wherein said electric heating elements (31') are arranged side by side in the length direction (X).

6. The continuous type heating pad of Claim 4, wherein said electric heating elements (31') are arranged side by side in a width direction (Y).

7. The continuous type heating pad of Claim 4, wherein said electric heating elements (31') have different configurations.

8. The continuous type heating pad of any one of Claims 1-7, wherein said laminated body is at least 3 m long.

9. The continuous type heating pad of any one of Claims 1-8, wherein said laminated body is rolled up to form a rolled laminated body (300), said rolled laminated body having a core (301), said laminated body being wound around said core.

10. The continuous type heating pad of Claim 9, which is produced by a process comprising:
advancing the first and second cover layers (10, 20) through a set of lamination rollers (40) and to a vulcanizing machine (100) including a vulcanizing drum (101);
moving an electric heating unit (30) between the first and second cover layers (10, 20) while the first and second cover layers (10, 20) are advanced through the lamination rollers (40);
bonding together the first and second cover layers (10, 20) to form the laminated body (200) by vulcanizing the first and second silicone rubber materials (120, 220) using the vulcanizing drum (101); and
rolling the laminated body (200) to form the rolled laminated body (300).
